# FASCICULE DE BREVET EUROPEEN

(11) **EP 2 198 924 B1**
(45) Date de publication et mention de la délivrance du brevet: **24.05.2017**
(21) Numéro de dépôt: 09179899.1
(22) Date de dépôt: 18.12.2009
(51) Int. Cl.: A61K 8/31, A61K 8/41, A61K 8/86, A61K 8/06, A61K 8/19, A61Q 5/06, A61Q 5/10, A61K 8/22

(54) **Procédé de coloration des matières keratiniques mettant en oeuvre une émulsion comprenant un colorant et un agent alcalin, et une composition oxydante**
VERFAHREN ZUR FÄRBUNG VON KERATINMATERIALIEN BEI VORHANDENSEIN EINER FARBSTOFF UND ALKALIWIRKSTOFFENTHALDENDEN EMULSION UND EINER OXIDATIONSZUSAMMENSETZUNG.
METHOD FOR DYEING KERATIN MATERIALS IN THE PRESENCE OF AN EMULSION COMPRISING A DYE AND AN ALKALINE AGENT, AND AN OXIDISING COMPOSITION

(30) Priorité: 19.12.2008 FR 0807322
(43) Date de publication de la demande: 23.06.2010
(73) Titulaire: L'Oréal, 75008 Paris (FR)
(72) Inventeur: Hercouet, Leïla, 93360, Neuilly Plaisance (FR); Bernard, Anne-Laure, 92160, Antony (FR); Bordeaux, Dominique, 91450, Soisy sur Seine (FR)
(74) Mandataire: Fevrier, Murielle Françoise E.

(56) Documents cités:
- EP-A- 0 424 261
- WO-A-02/089748
- DE-A1- 10 008 640
- FR-A- 1 517 715
- GB-A- 1 554 331
- US-A1- 2005 129 652
- US-A1- 2005 196 367
- US-A1- 2006 075 580

## Description

La présente invention a pour objet un procédé de coloration éclaircissante des matières kératiniques humaines, notamment les cheveux.

Les procédés d'éclaircissement des matières kératiniques telles que les fibres kératiniques humaines consistent à employer une composition aqueuse comprenant au moins un agent oxydant, en condition de pH alcalin dans la grande majorité des cas. Cet agent oxydant a pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

L'une des difficultés vient du fait que le procédé d'éclaircissement est mis en ouvre dans des conditions alcalines et que l'agent alcalin le plus couramment utilisé est l'ammoniaque. L'ammoniaque est particulièrement avantageux dans ce type de procédé. En effet, il permet d'ajuster le pH de la composition à un pH alcalin pour permettre l'activation de l'agent oxydant. Cet agent provoque également un gonflement de la fibre kératinique, avec un soulèvement des écailles, ce qui favorise la pénétration de l'oxydant à l'intérieur de la fibre et donc augmente l'efficacité de la réaction.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu (picotements).

Quant à l'option de purement et simplement remplacer en totalité ou en partie l'ammoniaque par un ou plusieurs autres agents alcalins classiques, celle-ci ne conduit pas à des compositions aussi efficaces que celles à base d'ammoniaque, notamment parce que ces agents alcalins ne conduisent pas un éclaircissement suffisant des fibres pigmentées en présence de l'agent oxydant.

Dans le cadre de la coloration des cheveux, une composition oxydante est utilisée pour colorer les cheveux de façon permanente à partir de précurseurs de colorants tels que les bases d'oxydations et les coupleurs. Dans le cadre de la coloration directe, bien que cette méthode ne nécessite pas l'emploi d'un agent oxydant pour développer la coloration, il n'est pas exclu d'en mettre en oeuvre afin d'obtenir un effet d'éclaircissement avec la coloration. On parle alors d'une coloration directe ou semi-permanente en conditions éclaircissantes.

Cet agent oxydant a pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

L'une des difficultés vient du fait que ces procédés sont mis en oeuvre dans des conditions alcalines et que l'agent alcalin le plus couramment utilisé est l'ammoniaque. L'emploi de l'ammoniaque est particulièrement avantageux dans ce type de procédés. En effet, il permet d'ajuster le pH de la composition à un pH alcalin pour permettre l'activation de l'agent oxydant. Mais cet agent alcalin provoque également un gonflement de la fibre kératinique, avec une ouverture des écailles, ce qui favorise la pénétration de l'oxydant, ainsi que des colorants, essentiellement les colorants d'oxydation, à l'intérieur de la fibre, et donc augmente l'efficacité de la réaction de coloration.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu se traduisant notamment par des picotements.

Quant à l'option de purement et simplement remplacer en totalité ou en partie l'ammoniaque par un ou plusieurs autres agents alcalinisants classiques, celle-ci ne conduit pas à des compositions aussi efficaces que celles à base d'ammoniaque, notamment parce que ces agents alcalinisants ne conduisent pas un éclaircissement suffisant des fibres pigmentées en présence de l'agent oxydant.

Ainsi l'un des objectifs de la présente invention est de proposer des procédés de coloration mis en oeuvre en présence d'un agent oxydant qui n'ont pas les inconvénients des procédés existants, tout en restant au moins aussi efficaces, tant sur le plan de la puissance de la coloration obtenue, que de la chromaticité, de l'homogénéité de la coloration le long de la fibre.

Ces buts et d'autres sont atteints par la présente invention qui a donc pour objet un procédé d'éclaircissement de matières kératiniques dans lequel on met en oeuvre :
a) une émulsion directe (A) comprenant un ou plusieurs corps gras liquides à température ambiante et à pression atmosphérique différents des acides gras en quantité supérieure à 25 % en poids, de préférence plus de 50 % le ou les corps gras étant choisis parmi les alcools gras, les esters d'acides gras, les esters d'alcool gras, les huiles minérales de plus de16 atomes de carbone, les huiles non siliconées végétales, animales ou synthétiques ; un ou plusieurs tensioactifs choisis parmi les tensioactifs non ioniques mono- ou poly- oxyalkylénés, mono- ou poly- glycérolés ; un ou plusieurs agents alcalins, choisis parmi les amines organiques, les sels d'amines organiques; une ou plusieurs espèces colorées ou colorantes choisies parmi les colorants directs et les colorants d'oxydation, et une quantité d'eau supérieure à 5 % et inférieure à 50% en poids, du poids total de l'émulsion,
b) une composition aqueuse (B) comprenant un ou plusieurs agents oxydants.

Elle concerne également un dispositif à plusieurs compartiments comprenant dans l'un d'eux une émulsion (A), dans un autre une composition (B) comprenant un ou plusieurs agents oxydants.

Dans le cadre de l'invention, une émulsion directe est une émulsion huile dans eau.

Dans ce qui va suivre, et à moins d'une autre indication, les bornes d'un domaine de valeurs sont comprises dans ce domaine.

Les matières kératiniques traitées par le procédé selon l'invention sont par exemple, les poils, les cils et les cheveux. Le procédé de l'invention permet notamment d'obtenir un bon niveau d'éclaircissement de ces matières kératiniques telles que les cheveux sans dégagement d'une odeur d'ammoniaque, pouvant être irritante.

L'émulsion (A) présente plus particulièrement une teneur en eau inférieure à 50 % en poids, de préférence comprise entre 10 et 50 % en poids, par rapport au poids de l'émulsion.

L'émulsion huile dans eau utile dans la présente invention comprend un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). En outre, les corps gras sont généralement solubles dans les solvants organiques dans les mêmes conditions de température et de pression, comme par exemple le chloroforme, l'éthanol , le benzène ou le décaméthylcyclopentasiloxane.

Selon la présente invention, la composition comprend au moins 25 % d'un ou plusieurs corps gras différents des acides gras.

Il est rappelé qu'au sens de l'invention, les alcools, esters et acides gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

Comme huiles non siliconées utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les hydrocarbures de plus de 16 atomes de carbone linéaires ou ramifiés, d'origine minérale ou synthétique, tels que les huiles de paraffine, la vaseline, l'huile de vaseline, les polydécènes, le polyisobutène hydrogéné tel que Parléam®.
- les huiles fluorées comme le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras utilisables dans la composition de l'invention sont non oxyalkylénés. Ils sont saturés ou insaturés, linéaires ou ramifiés, et comportent 6 à 30 atomes de carbone et plus particulièrement de 8 à 30 atomes de carbone.

Les esters sont les esters de mono ou polyacides aliphatiques saturés ou insaturés, linéaires ou ramifiés en C1-C26 et de mono ou polyalcools aliphatiques saturés ou insaturés, linéaires ou ramifiés en C1-C26, le nombre total de carbone des esters étant plus particulièrement supérieur ou égal à 10.

Toujours dans le cadre de cette variante, on peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.

La composition peut également comprendre, à titre d'ester gras, des esters et diesters de sucres d'acides gras en C6-C30, de préférence en C12-C22. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C6-C30, de préférence en C12-C22, linéaires ou ramifiés, saturés ou insaturés. S'ils sont insaturés, ces composés peuvent comprendre une à trois double-liaisons carbone-carbone, conjuguées ou non.

De préférence, les corps gras ne sont ni oxyalkylénés, ni glycérolés. L'émulsion (A) selon l'invention comprend au moins 25 % de corps gras. De préférence la concentration en corps gras va de 25 à 80 % ,encore plus préférentiellement de 25 à 65 %, mieux de 30 à 55 % du poids total de l'émulsion (A). L'émulsion (A) comprend également un ou plusieurs tensioactifs.

Les tensioactifs sont choisis parmi les tensioactifs non ioniques mono ou poly- oxyalkylénés, et mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentant un nombre de moles d'oxyde d'éthylène et/ou de propylène compris entre 1 et 100, de préférence entre 2 et 50. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀ oxyéthylénés., de préférence C₁₈-C₃₀ oxyéthylénés.

Comme alcools gras éthoxylés, on peut citer par exemple les produits d'addition d'oxyde d'éthylène avec l'alcool laurylique, notamment ceux comportant de 9 à 50 groupes oxyéthylénés et plus particulièrement ceux comportant de 10 à 12 groupes oxyéthylénés (Laureth-10 à Laureth-12 en noms CTFA) ; les produits d'addition d'oxyde d'éthylène avec l'alcool béhénylique, notamment ceux comportant de 9 à 50 groupes oxyéthylénés (Beheneth-9 à Beheneth-50 en noms CTFA) ; les produits d'addition d'oxyde d'éthylène avec l'alcool cétéarylique (mélange d'alcool cétylique et d'alcool stéarylique), notamment ceux comportant de 10 à 30 groupes oxyéthylénés (Ceteareth-10 à Ceteareth-30 en noms CTFA); les produits d'addition d'oxyde d'éthylène avec l'alcool cétylique, notamment ceux comportant de 10 à 30 groupes oxyéthylénés (Ceteth-10 à Ceteth-30 en noms CTFA) ; les produits d'addition d'oxyde d'éthylène avec l'alcool stéarylique, notamment ceux comportant de 10 à 30 groupes oxyéthylénés (Steareth-10 à Steareth-30 en noms CTFA); les produits d'addition d'oxyde d'éthylène avec l'alcool isostéarylique, notamment ceux comportant de 10 à 50 groupes oxyéthylénés (Isosteareth-10 à Isosteareth-50 en noms CTFA) ; et leurs mélanges.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA : PEG-9 palmitate à PEG-50 palmitate) ; les stéarates de PEG-9 à PEG-50 (en noms CTFA : PEG-9 stearate à PEG-50 stearate) ; les palmito-stéarates de PEG-9 à PEG-50 ; les béhénates de PEG-9 à PEG-50 (en noms CTFA : PEG-9 behenate à PEG-50 behenate) ; et leurs mélanges.

On peut utiliser aussi des mélanges de ces dérivés oxyéthylénés d'alcools gras et d'acides gras.

Selon un mode préféré de réalisation, l'émulsion (A) comprend au moins un alcool gras éthoxylé, et de préférence au moins l'alcool béhénylique.

A titre d'exemple de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCl : POLYGLYCERYL-4 LAURYL ETHER), l'alcool laurique à 1,5 moles de glycérol, l'alcool oléique à 4 moles de glycérol (nom INCl : POLYGLYCERYL-4 OLEYL ETHER), l'alcool oléique à 2 moles de glycérol (Nom INCl : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

De préférence, le tensioactif présent dans l'émulsion est un tensioactif non ionique présentant un HLB de 8 à 18. Le HLB est le rapport entre la partie hydrophile et la partie lipophile dans leur molécule. Ce terme HLB est bien connu de l'homme du métier et est décrit dans "The HLB system. A time-saving guide to Emulsifier Selection" (published by ICI Americas Inc ; 1984).

La teneur en tensioactifs dans l'émulsion (A) représente plus particulièrement de 0,1 à 50 % en poids, de préférence de 0,5 à 30 % en poids par rapport au poids de la composition anhydre.

L'émulsion (A) peut être préparée par des procédés de préparation classique d'émulsion directe mais aussi par un procédé par PIT. Le principe d'émulsification par inversion de phase en température (en Anglais : Phase Inversion Temperature ou PIT) est, dans son principe, bien connu de l'homme de l'art ; il a été décrit en 1968 par K. Shinoda (J. Chem. Soc. Jpn., 1968, 89, 435). Il a été montré que cette technique d'émulsification permet d'obtenir des émulsions fines stables (K. Shinoda et H. Saito, J. Colloïd Interface Sci., 1969,30, 258). Cette technologie a été appliquée en cosmétique dès 1972 par Mitsui et al. («Application of the phase-inversion-temperature method to the emulsification of cosmetics» ; T. Mitsui, Y. Machida and F. Harusawa, American. Cosmet. Perfum., 1972, 87, 33).

Le principe de cette technique est le suivant : on réalise le mélange d'une phase aqueuse et d'une phase huileuse, que l'on porte à une température supérieure à la température PIT, température d'inversion de phase du système, qui est la température à laquelle l'équilibre entre les propriétés hydrophile et lipophile du ou des émulsionnants mis en oeuvre est atteint ; à température élevée, c'est-à-dire supérieure à la température d'inversion de phase (>PIT), l'émulsion est de type eau-dans-huile, et, au cours de son refroidissement, cette émulsion s'inverse à la température d'inversion de phase, pour devenir une émulsion de type huile-dans-eau, et ceci en étant passée auparavant par un état de microémulsion. Selon ce mode de préparation particulier de l'émulsion, au moins un des tensio-actifs de l'émulsion est un tensioactif nonionique de HLB compris entre 8 et 18, de préférence un tensioactif non ionique oxyalkyléné, notamment oxyéthyléné. Ce procédé permet d'obtenir facilement des émulsions de diamètre inférieur à 4 µm, de préférence inférieure à 1µm.

L'émulsion utile dans la présente invention comprend un ou plusieurs agents alcalins.

L'agent alcalin est choisi parmi les amines organiques, les sels d'amines organiques, seuls ou en mélange.

A titre d'exemple d'amine organique, on peut citer les amines organiques dont le pKb à 25°C est inférieur à 12, et de préférence inférieur à 10, encore plus avantageusement inférieur à 6. Il est à noter qu'il s'agit du pKb correspondant à la fonction de basicité la plus élevée.

L'amine organique peut comprendre une ou deux fonctions amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les amines organiques choisies parmi les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Conviennent également les amines organiques de formule suivante : 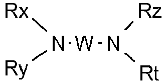 dans laquelle W est un reste alkylène en C₁-C₆ éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles amines, le 1,3 diaminopropane, le 1,3 diamino 2 propanol, la spermine, la spermidine.

Selon une autre variante de l'invention, l'amine organique est choisie parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (I) suivante : 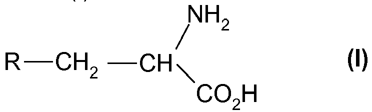 où R désigne un groupe choisi parmi :

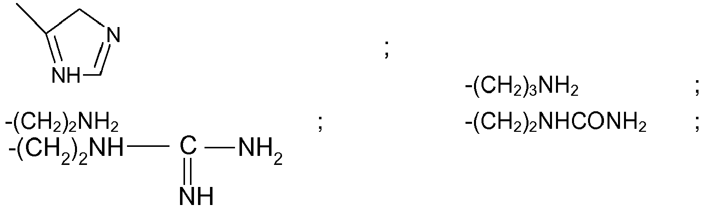

Les composés correspondants à la formule (I) sont l'histidine, la lysine, l'arginine, l'ornithine, la citrulline.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

Selon une variante préférée de l'invention, l'amine organique est choisie parmi les acides aminés basiques. Les acides aminés particulièrement préférés sont l'arginine, la lysine, l'histidine, ou leurs mélanges.

Selon une autre variante de l'invention, l'amine organique est choisie parmi les amines organiques de type hétérocycliques On peut en particulier citer, outre l'histidine déjà mentionnée dans les acides aminés, la pyridine, la pipéridine, l'imidazole, le 1,2,4-triazole, le tétrazole, le benzimidazole .

Selon une autre variante de l'invention, l'amine organique est choisie parmi les dipeptides d'acides aminés. A titre de dipeptides d'acides aminés utilisables dans la présente invention, on peut notamment citer la carnosine, l'anserine et la baleine

Selon une autre variante de l'invention, l'amine organique est choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl]amino)ethane-1-sulfonique.

De préférence l'amine organique est une alcanolamine. Plus préférentiellement l'amine organique est choisie parmi le 2-amino 2-méthyl 1-propanol, la monoéthanolamine ou leurs mélanges. Encore plus préférentiellement l'amine organique est la monoéthanolamine.

L'agent alcalin peut être une amine organique sous forme de sels. Par sel d'amine organique, on entend au sens de la présente invention, les sels organiques ou inorganiques d'une amine organique telle que décrite ci-dessus.

De préférence, les sels organiques sont choisis parmi les sels d'acides organiques tels que les citrates, les lactates, les glycolates, les gluconates, les acétates, les propionates, les fumarates, les oxalates et les tartrates.

De préférence, les sels inorganiques sont choisis parmi les halogénohydrates (chlorhydrates par exemple), les carbonates, les hydrogénocarbonates, les sulfates, les hydrogénophosphates et les phosphates.

Par composé inorganique, au sens de la présente invention, on entend tout composé possédant dans sa structure un ou plusieurs éléments des colonnes 1 à 13 du tableau périodique des éléments autre que l'hydrogène, ne comportant pas simultanément d'atome(s) de carbone et d'hydrogène.

Selon un mode de réalisation particulier de l'invention, la base inorganique contient un ou plusieurs éléments des colonnes 1 et 2 du tableau périodique des éléments autre que l'hydrogène.

Dans une variante préférée la base inorganique présente la structure suivante :

(Z₁^{x-})ₘ(Z₂^{y+})ₙ

dans laquelle
Z₂ désigne un métal des colonnes 1 à 13 du tableau périodique des éléments, de préférence 1 ou 2, comme le sodium ou le potassium ;
Z₁^{x-} désigne un anion choisi parmi les ions CO₃²⁻, OH⁻, HCO₃²⁻, SiO₃²⁻, HPO₄²⁻, PO₄³⁻, B₄O₇²⁻, de préférence parmi les ions CO₃²⁻, OH⁻, SiO₃²⁻ ;
x désigne 1 , 2 ou 3 ;
y désigne 1, 2, 3 ou 4 ;
m et n désignent indépendamment l'un de l'autre 1, 2, 3 ou 4 ;
avec n.y=m.x.

De préférence, la base inorganique correspond à la formule suivante (Z₁^{x-}) ₘ(Z₂^{y+})ₙ, dans laquelle Z₂ désigne un métal des colonnes 1 et 2, du tableau périodique des éléments ; Z₁^{x-} désigne un anion choisi parmi les ions CO₃²⁻, OH⁻, SiO₃²⁻, x vaut 1, y désigne 1 ou 2, m et n désignent indépendamment l'un de l'autre 1 ou 2 avec n.y=m.x.

A titre de base inorganique utilisable selon l'invention on peut citer, le bicarbonate de sodium, le carbonate de potassium, la soude, la potasse, le métasilicate de sodium, le métasilicate de potassium.

On peut aussi utiliser en tant qu'agent alcalin des sels d'ammoniums.

Les sels d'ammonium utilisables dans la composition B selon la présente invention sont les sels d'ammonium (NH4+).

Les sels d'ammonium utilisables dans la composition B selon la présente invention sont de préférence choisi parmi les sels d'acide suivants : acétate, carbonate, bicarbonate, chlorure, citrate, nitrate, nitrite, phosphate, sulfate. De manière particulièrement préférée, le sel est le carbonate tel que le carbonate d'ammonium.

Selon un mode de réalisation particulier, la composition contient en tant qu'agents alcalins au moins une amine organique, de préférence au moins une alkanolamine. Lorsque la composition contient plusieurs agents alcalins dont une alkanolamine et de l'ammoniaque ou un de ses sels, la ou les amines organiques sont de préférence majoritaire en poids par rapport à la quantité ammoniac.

Généralement, l'émulsion (A) présente une teneur en agents alcalins allant de 0,1 à 40 % en poids, de préférence de 0,5 à 20 % en poids par rapport au poids de ladite composition.

L'émulsion (A) comprend une ou plusieurs espèces colorantes ou colorées choisies parmi les colorants d'oxydation et les colorants directs.

Les colorants d'oxydation sont en général choisis parmi les bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-((β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-((β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one,4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

L'emulsion (A) selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10 % en poids par rapport au poids total de la composition, et de préférence de 0,005 à 5 % en poids par rapport au poids total de la composition.

La teneur en coupleur(s), s'il(s) est(sont) présent(s), représentent chacun avantageusement de 0,0001 à 10 % en poids par rapport au poids total de la composition, et de préférence de 0,005 à 5 % en poids par rapport au poids total de la composition cosmétique (B).

En ce qui concerne les colorants directs, ces derniers sont plus particulièrement choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Plus particulièrement, les colorants azoïques comprennent une fonction - N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine, azométhine, mono- et diarylméthane, indoamines (ou diphénylamines), indophénols, indoanilines, carbocyanines, azacabocyanines et leurs isomères, diazacarbocyanines et leurs isomères, tétraazacarbocyanines, hémicyanines

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges.

Ces colorants peuvent être des colorants monochromophoriques (c'est-à-dire ne comprenant qu'un seul colorant) ou polychromophoriques, de préférence di- ou tri- chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

Parmi les colorants directs benzéniques utilisables selon l'invention, on peut citer de manière non limitative les composés suivants :
- 1,4-diamino-2-nitrobenzène,
- 1-amino-2 nitro-4-ß-hydroxyéthylaminobenzène
- 1-amino-2 nitro-4-bis(β-hydroxyéthyl)-aminobenzène
- 1,4-bis(ß-hydroxyéthylamino)-2-nitrobenzène
- 1-ß-hydroxyéthylamino-2-nitro-4-bis-(β-hydroxyéthylamino)-benzène
- 1-ß-hydroxyéthylamino-2-nitro-4-aminobenzène
- 1-β-hydroxyéthylamino-2-nitro-4-(éthyl)(ß-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR2189006, FR 2285851, FR-2140205, EP 1378544, EP 1674073.

Parmi cela, on peut aussi citer les composés suivants :

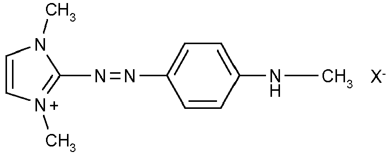

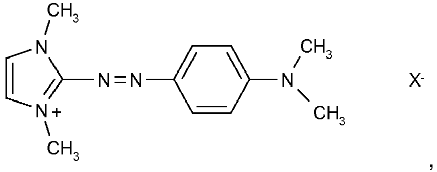

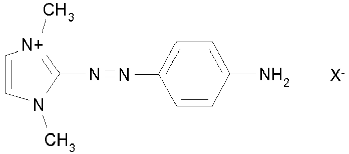

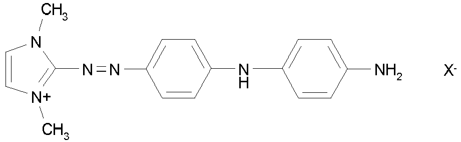

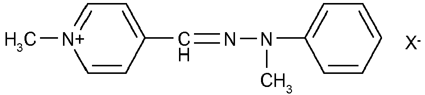

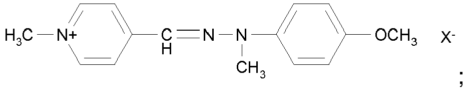

On peut également citer parmi les colorants directs azoïques les colorants suivants, décrits dans le COLOUR INDEX INTERNATIONAL 3e édition :
- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :
- Disperse Red 15
- Solvent Violet 13
- Disperse Violet 1
- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99
ainsi que les composés suivants :
- 1-N-méthylmorpholiniumpropylamino-4-hydroxy anthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous, An étant défini comme précédemment :

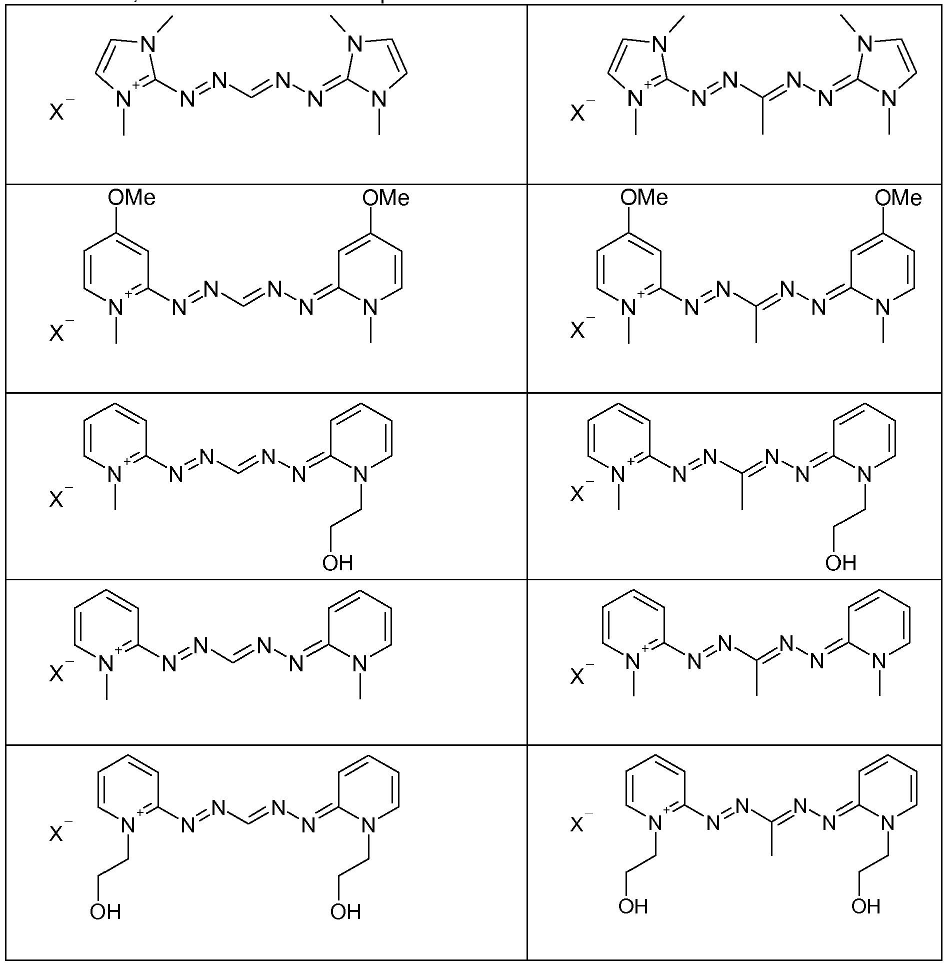

X représente un anion de préférence choisi parmi le chlorure, l'iodure, le méthyl sulfate, l'éthyl sulfate, l'acétate et le perchlorate.

A titre d'exemples de colorants polychromophoriques, on pourra notamment se reporter aux demandes de brevets EP 1637566, EP 1619221, EP 1634926, EP 1619220, EP 1672033, EP 1671954, EP 1671955, EP 1679312, EP 1671951, EP167952, EP167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent plus particulièrement de 0,0001 à 10 % en poids du poids total de la composition, et de préférence de 0,005 à 5 % en poids.

L'émulsion (A) peut comprendre l'un et ou l'autre types de colorants. Elle peut éventuellement être issue du mélange de deux compositions colorantes l'une comprenant le ou les colorants d'oxydation, l'autre, le ou les colorants directs.

L'émulsion (A) peut également renfermer divers adjuvants utilisés classiquement dans les compositions pour l'éclaircissement des cheveux, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères ; des agents antioxydants ; des agents de pénétration ; des agents séquestrants ; des parfums ; des agents dispersants ; des agents filmogènes ; des agents conservateurs ; des agents opacifiants.

Elle peut éventuellement comprendre un ou plusieurs solvants organiques. A titre de solvant organique, on peut par exemple citer, les alcanols, linéaires ou ramifiés, en C₂-C₄, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le glycérol, le monoéthyléther et le monométhyléther du diéthylèneglycol, ainsi que les alcools aromatiques comme l'alcool benzylique ou le phénoxyéthanol, et leurs mélanges.

Le procédé est mis en oeuvre avec une composition (B) comprenant un ou plusieurs agents oxydants.

Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates, les peracides et leurs précurseurs et les percarbonates de métaux alcalins ou alcalino-terreux.

Cet agent oxydant est avantageusement constitué par du peroxyde d'hydrogène et notamment en solution aqueuse (eau oxygénée) dont le titre peut varier, plus particulièrement, de 1 à 40 volumes (soit 0.3 à 12% de H2O2), et encore plus préférentiellement de 5 à 40 volumes (soit 1.5 à 12% de H2O2).

En fonction du degré d'éclaircissement souhaité, l'agent oxydant peut également comprendre un agent oxydant choisi de préférence parmi les sels peroxygénés.

La composition (B) est généralement une composition aqueuse. Par composition aqueuse, on entend une composition comprenant plus de 5 % en poids d'eau, de préférence plus de 10 % en poids d'eau, et de manière encore plus avantageuse plus de 20 % en poids d'eau

Cette composition (B) peut également comprendre un ou plusieurs solvants organiques tels que décrits précédemment. Elle peut aussi comprendre un ou plusieurs agents acidifiants.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition (B), est inférieur à 7.

Enfin, la composition (B) se présente sous diverses formes, comme par exemple une solution, une émulsion ou un gel.

Le procédé de l'invention peut être mis en oeuvre en appliquant l'émulsion (A) et la composition (B) successivement et sans rinçage intermédiaire.

Selon une autre variante, on applique sur les matières kératiniques, sèches ou humides, une composition obtenue par mélange extemporané, au moment de l'emploi, de l'émulsion (A) et de la composition (B). Selon ce mode de réalisation, le rapport pondéral des quantités de (A) / (B) varie de 0,1 à 10 de préférence de 0,2 à 2, mieux de 0,3 à 1.

En outre, indépendamment de la variante mise en oeuvre, le mélange présent sur les matières kératiniques (résultant soit du mélange extemporané de (A) et (B) ou de leur application successive partielle ou totale) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les matières kératiniques sont éventuellement rincées à l'eau, subissent éventuellement un lavage suivi d'un rinçage à l'eau, avant d'être séchées ou laissées à sécher.

Dans une variante préférée de l'invention, les matières kératiniques sont des fibres kératiniques tels que les poils, cils et cheveux.

Selon une variante, la composition de coloration obtenue après mélange de l'émulsion (A) décrite précédemment et de la composition aqueuse (B) comprenant un agent oxydant est telle que après mélange la quantité de corps gras est supérieure à 20 %, de préférence supérieure à 25 %, voire supérieure à 30 %.

Enfin, l'invention concerne un dispositif à plusieurs compartiments comprenant dans un premier compartiment une émulsion (A), et dans un second, une composition aqueuse (B) comprenant un ou plusieurs agents oxydants, ces compositions ayant été décrites auparavant.

### EXEMPLES

### L'émulsion A1 suivante a été préparée selon un procédé d'inversion de phase en température (procédé PIT)

### Procédé de fabrication

- On chauffe la phase A au bain marie sous rayneri (400 tr/min). On obtient une émulsion blanche fluide qui devient translucide vers 68°C (passage par une phase de microémulsion) et s'épaissit au-delà.
- Dès que l'émulsion s'épaissit, on retire le bain marie : on laisse refroidir sous la même agitation.
- Vers 50°C, on introduit le poloxamer.
- A Température ambiante, on introduit l'éthanol, la monoéthanolamine, le bicarbonate de potassium, la base et le coupleur préalablement dispersé dans 4,302 g d'eau et on réajuste l'eau perdue à l'évaporation (<5%).
On obtient ainsi une émulsion gélifiée translucide de taille de gouttes < 1 µm (viscosité 8UD M4, taille des goûtes < 1µm, pH =11.3)

| **Emulsion A1** | | |
|---|---|---|
| **Phase** | **Nom INCI** | **g%** |
| A | Beheneth-10 | 6,00 |
| | Sorbitol | 5,00 |
| | Huile de vaseline | 60,25 |
| | Eau | 10,00 |
| B | Ethanol | 2,00 |
| | Poloxamer 184 | 5,00 |
| | Bicarbonate de potassium | 1,75 |
| | Eau | 4,302 |
| | Monoéthanolamine | 5,00 |
| | p-phenylenediamine | 0,216 |
| | Dichlorhydrate de 2,4-diaminophenoxyéthanol | 0,482 |

Au moment de l'emploi, on mélange 1 poids l'émulsion A1 à 1,5 poids d'une composition aqueuse (B1) oxydante comprenant une dispersion d'alcools gras dans l'eau (8%) et 6% de peroxyde d'hydrogène : Platinium 20V .

Le mélange est ensuite appliqué sur une mèche de cheveux naturels à 90% de cheveux blancs (hauteur de ton = 4). Le rapport de bain « mélange/mèche » est respectivement de 10/1 (g/g). Le temps de pose est de 30min à 27°C. A l'issue de ce temps, les mèches sont rincées, puis lavées avec du shampooing Elsève multivitamines.

On obtient une coloration bleue puissante et sans odeur.

## Revendications

1. Procédé de coloration de matières kératiniques dans lequel on met en oeuvre :
a) une émulsion directe (A) comprenant un ou plusieurs corps gras liquides à température ambiante et à pression atmosphérique différents des acides gras en quantité supérieure à 25 % en poids, le ou les corps gras étant choisis parmi les alcools gras, les esters d'acide gras, les esters d'alcool gras, les huiles minérales de plus de 16 atomes de carbone, les huiles non siliconées végétales, animales ou synthétiques ; un ou plusieurs tensioactifs choisis parmi les tensioactifs non ioniques mono- ou poly- oxyalkylénés, mono- ou poly- glycérolés ; un ou plusieurs agents alcalins choisis parmi les amines organiques, les sels d'amines organiques, une ou plusieurs espèces colorées ou colorantes choisies parmi les colorants directs et les colorants d'oxydation, et une quantité d'eau supérieure à 5 % et inférieure à 50 % en poids, du pois total de l'émulsion,
b) une composition (B) comprenant un ou plusieurs agents oxydants.

2. Procédé selon la revendication précédente, **caractérisé en ce que** l'émulsion (A) comprend plus de 50 % en poids de corps gras.

3. Procédé selon la revendication 1 ou 2 dans lequel la teneur en eau dans l'émulsion (A) est supérieure à 10 % en poids.

4. Procédé selon l'une quelconque des revendications précédentes, **caractérisé en ce que** la teneur en corps gras est comprise entre 25 et 80% en poids par rapport au poids de l'émulsion (A).

5. Procédé selon l'une quelconque des revendications précédentes, **caractérisé en ce que** l'amine organique est une alcanolamine, de préférence choisie parmi le 2-amino 2-méthyl 1-propanol, la monoéthanolamine ou leurs mélanges, un acide aminé basique choisi parmi l'arginine, l'histidine, la lysine, ou leurs mélanges.

6. Procédé selon l'une quelconque des revendications précédentes dans lequel le ou les colorants d'oxydation sont choisis parmi les paraphénylènediamines, les bis-phénylalkylènediamines, les para-aminophénols, les ortho-aminophénols, les bases hétérocycliques et leurs sels d'addition, les métaphénylènediamines, les méta-aminophénols, les méta-diphénols, les coupleurs naphtaléniques, les coupleurs hétérocycliques ainsi que leurs sels d'addition.

7. Procédé selon l'une quelconque des revendications précédentes, **caractérisé en ce que** la composition (B) comprend un ou plusieurs agents oxydants choisis parmi le peroxyde d'hydrogène, le peroxyde d'urée, les bromates ou ferricyanures de métaux alcalins, les sels peroxygénés comme par exemple les persulfates, les perborates, les peracides et leurs précurseurs, et les percarbonates de métaux alcalins ou alcalino-terreux, de préférence le peroxyde d'hydrogène.

8. Procédé selon l'une quelconque des revendications précédentes dans lequel la composition (B) comprend plus de 5 % en poids d'eau, de préférence plus de 20 %.

9. Procédé selon l'une quelconque des revendications précédentes, **caractérisé en ce que** l'on applique sur les fibres kératiniques, une composition obtenue par mélange extemporané, au moment de l'emploi, de l'émulsion (A) et de la composition (B).

10. Procédé selon l'une quelconque des revendications 1 à 9, **caractérisé en ce que** l'on applique sur les fibres kératiniques, successivement et sans rinçage intermédiaire, l'émulsion (A) et la composition (B).

11. Dispositif à plusieurs compartiments comprenant dans un premier compartiment, l'émulsion (A) selon l'une des revendications 1 à 6, dans un autre compartiment une composition (B) telle que définie à l'une des revendications 1 et 7 à 8.

## Patentansprüche

1. Verfahren zum Färben von Keratinsubstanzen, bei dem man Folgendes einsetzt:
a) eine direkte Emulsion (A), umfassend ein oder mehrere bei Umgebungstemperatur und Atmosphärendruck flüssige Fette, die keine Fettsäuren sind, in einer Menge von mehr als 25 Gew.-% umfasst, wobei das oder die Fett(e) aus Fettalkoholen, Fettsäureestern, Fettalkoholestern, Mineralölen mit mehr als 16 Kohlenstoffatomen, nicht-silikonisierten pflanzlichen, tierischen oder synthetischen Ölen ausgewählt ist/sind; ein oder mehrere Tenside, die aus nichtionischen mono- oder polyoxyalkylenierten, mono- oder polyglycerinierten Tensiden ausgewählt sind; ein oder mehrere alkalische Agentien, die aus organischen Aminen, organischen Aminsalzen ausgewählt sind, einen oder mehrere farbige (n) oder färbende (n) Stoff(e), die aus Direktfarbstoffen und Oxidationsfarbstoffen ausgewählt sind, und eine Menge an Wasser von mehr als 5 Gew.-% und weniger als 50 Gew.-%, bezogen auf das Gesamtgewicht der Emulsion,
b) eine Zusammensetzung (B), die ein oder mehrere Oxidationsmittel umfasst.

2. Verfahren nach dem vorhergehenden Anspruch, **dadurch gekennzeichnet, dass** die Emulsion (A) mehr als 50 Gew.-% Fett umfasst.

3. Verfahren nach Anspruch 1 oder 2, wobei der Gehalt an Wasser in der Emulsion (A) größer als 10 Gew.-% ist.

4. Verfahren nach einem der vorhergehenden Ansprüche, **dadurch gekennzeichnet, dass** der Gehalt an Fett zwischen 25 Gew.-% und 80 Gew.-%, bezogen auf das Gewicht der Emulsion (A), beträgt.

5. Verfahren nach einem der vorhergehenden Ansprüche, **dadurch gekennzeichnet, dass** das organische Amin ein Alkanolamin ist, das vorzugsweise aus 2-Amino-2-methyl-1-propanol, Monoethanolamin oder deren Gemischen, einer aus Arginin, Histidin und Lysin oder deren Gemischen ausgewählten basischen Aminosäure ausgewählt wird.

6. Verfahren nach einem der vorhergehenden Ansprüche, wobei der oder die Oxidationsfarbstoff(e) aus para-Phenylendiaminen, Bisphenylalkylendiaminen, para-Aminophenolen, ortho-Aminophenolen, heterozyklischen Basen und deren Additionssalzen, meta-Phenylendiaminen, meta-Aminophenolen, meta-Diphenolen, Naphthalin-Kupplern, heterozyklischen Kupplern sowie deren Additionssalzen ausgewählt ist/sind.

7. Verfahren nach einem der vorhergehenden Ansprüche, **dadurch gekennzeichnet, dass** die Zusammensetzung (B) ein oder mehrere, aus Wasserstoffperoxid, Harnstoffperoxid, Alkalimetallbromaten und -ferricyaniden, peroxygenierten Salzen, wie zum Beispiel Persulfaten, Perboraten, Persäuren und deren Vorläufern und Alkalimetall- oder Erdalkalimetallpercarbonaten, vorzugsweise Wasserstoffperoxid, ausgewähltes Oxidationsmittel umfasst.

8. Verfahren nach einem der vorhergehenden Ansprüche, wobei die Zusammensetzung (B) mehr als 5 Gew.-% Wasser, vorzugsweise mehr als 20 Gew.-%, umfasst.

9. Verfahren nach einem der vorhergehenden Ansprüche, **dadurch gekennzeichnet, dass** man auf die Keratinfasern eine Zusammensetzung, die durch bedarfsgemäßes Mischen der Emulsion (A) und der Zusammensetzung (B) unmittelbar vor der Verwendung erhalten wird, aufbringt.

10. Verfahren nach einem der Ansprüche 1 bis 9, **dadurch gekennzeichnet, dass** man auf die Keratinfasern nacheinander und ohne Zwischenspülung die Emulsion (A) und die Zusammensetzung (B) aufbringt.

11. Vorrichtung mit mehreren Abteilungen, die in einer ersten Abteilung die Emulsion (A) nach einem der Ansprüche 1 bis 6 und in einer anderen Abteilung eine Zusammensetzung (B) nach einem der Ansprüche 1 und 7 bis 8 umfasst.

## Claims

1. Process for dyeing keratin materials, in which the following are employed:
a) a direct emulsion (A) comprising one or more fatty substances that are liquid at room temperature and at atmospheric pressure, other than the fatty acids, in an amount of greater than 25% by weight, the fatty substance(s) being chosen from fatty alcohols, fatty acid esters, fatty alcohol esters, mineral oils containing more than 16 carbon atoms, non-silicone-based plant, animal or synthetic oils; one or more surfactants chosen from monooxyalkylenated or polyoxyalkylenated, or monoglycerolated or polyglycerolated, nonionic surfactants; one or more alkaline agents chosen from organic amines, salts of organic amines, one or more coloured or colouring species chosen from direct dyes and oxidation dyes, and an amount of water greater than 5% and less than 50% by weight, of the total weight of the emulsion,
b) a composition (B) comprising one or more oxidizing agents.

2. Process according to the preceding claim, **characterized in that** the emulsion (A) comprises more than 50% by weight of fatty substance.

3. Process according to Claim 1 or 2, in which the content of water in the emulsion (A) is greater than 10% by weight.

4. Process according to any one of the preceding claims, **characterized in that** the content of fatty substance is between 25 and 80% by weight relative to the weight of the emulsion (A).

5. Process according to any one of the preceding claims, **characterized in that** the organic amine is an alkanolamine, preferably chosen from 2-amino-2-methyl-1-propanol or monoethanolamine or mixtures thereof, a basic amino acid chosen from arginine, histidine, lysine or mixtures thereof.

6. Process according to any one of the preceding claims, in which the oxidation dye(s) are chosen from para-phenylenediamines, bis(phenyl)alkylenediamines, para-aminophenols, ortho-aminophenols, heterocyclic bases and the addition salts thereof, metaphenylenediamines, meta-aminophenols, meta-diphenols, naphthalene couplers, heterocyclic couplers, and the addition salts thereof.

7. Process according to any one of the preceding claims, **characterized in that** the composition (B) comprises one or more oxidizing agents chosen from hydrogen peroxide, urea peroxide, alkali metal bromates or ferricyanides, peroxygenated salts, such as, for example, persulfates, perborates, peracids and precursors thereof, and alkali metal or alkaline earth metal percarbonates, preferably hydrogen peroxide.

8. Process according to any one of the preceding claims, in which the composition (B) comprises more than 5% by weight of water, preferably more than 20%.

9. Process according to any one of the preceding claims, **characterized in that** a composition obtained by extemporaneous mixing, at the time of use, of the emulsion (A) and the composition (B), is applied to the keratin fibres.

10. Process according to any one of Claims 1 to 9, **characterized in that** the emulsion (A) and the composition (B) are applied to the keratin fibres successively and without intermediate rinsing.

11. Multi-compartment device comprising, in a first compartment, the emulsion (A) according to one of Claims 1 to 6, in another compartment a composition (B) as defined in one of Claims 1, 7 and 8.
